# EUROPEAN PATENT SPECIFICATION

(11) **EP 2 878 592 B1**
(45) Date of publication and mention of the grant of the patent: **20.02.2019**
(21) Application number: 14188019.5
(22) Date of filing: 07.10.2014
(51) Int. Cl.: C07C 235/60, C07D 263/57, C07C 237/20, C07D 491/052, C07D 213/75, C07D 213/80, C07D 333/22, C07D 333/24, C07C 317/44, C07C 233/22, C07C 233/60, C07C 235/34, C07D 263/32, A61K 31/165, A61P 29/00, C07C 235/78, C07C 237/30, C07C 237/44, C07D 207/20, C07C 245/08, C07D 207/337, C07D 209/28, C07D 209/46, A61K 31/40, A61K 31/381, A61K 31/421, A61K 31/655

(54) **SYNTHESIS OF AMIDE DERIVATIVES OF SOME NONSTEROIDAL ANTIINFLAMMATORY DRUGS AS POTENTIAL PRO-DRUGS**
SYNTHESE VON AMIDDERIVATEN EINIGER NICHTSTEROIDALER ENTZÜNDUNGSHEMMENDER MEDIKAMENTE ALS POTENZIELLE PRODRUGS
SYNTHÈSE DE DÉRIVÉS D'AMIDE DE CERTAINS MÉDICAMENTS ANTI-INFLAMMATOIRES NON STÉROÏDIENS EN TANT QUE PROMÉDICAMENTS POTENTIELS

(30) Priority: 27.11.2013 TR 201313845
(43) Date of publication of application: 03.06.2015
(73) Proprietor: Kücükgüzel, Ilkay, Istanbul (TR)
(72) Inventor: KÜCÜKGÜZEL, Ilkay, Istanbul (TR); KULABAS, Necla, Istanbul (TR); SET, Irem, 41100 Istanbul (TR)
(74) Representative: Mutlu, Aydin

(56) References cited:
- EP-A1- 2 418 200
- RAJAT K. DAS ET AL: "Spectroscopic, microscopic and first rheological investigations in charge-transfer interaction induced organogels", JOURNAL OF MATERIALS CHEMISTRY, vol. 20, no. 34, 1 January 2010 (2010-01-01), page 7227, XP055177568, ISSN: 0959-9428, DOI: 10.1039/c0jm01192d -& Rajat K Das ET AL: "Supporting Information Spectroscopic, Microscopic and first Rheological investigations in Charge- transfer interaction induced Organogels", The Royal Society of Chemistry, 1 January 2010 (2010-01-01), XP055177502, Retrieved from the Internet: URL:http://www.rsc.org/suppdata/jm/c0/c0jm 01192d/c0jm01192d.pdf [retrieved on 2015-03-18]
- PIERA SOZIO ET AL: "Ibuprofen and Lipoic Acid Diamides as Potential Codrugs with Neuroprotective Activity", ARCHIV DER PHARMAZIE, WILEY VERLAG, WEINHEIM, vol. 343, no. 3, 1 March 2010 (2010-03-01) , pages 133-142, XP002649901, ISSN: 0365-6233, DOI: 10.1002/ARDP.200900152 [retrieved on 2010-02-23]
- MICHAEL BRANDL ET AL: "Approaches for improving the stability of ketorolac in powder blends", JOURNAL OF PHARMACEUTICAL SCIENCES, vol. 84, no. 10, 1 October 1995 (1995-10-01), pages 1151-1153, XP055177263, ISSN: 0022-3549, DOI: 10.1002/jps.2600841003
- CHEN YAO ET AL: "NO-donating tacrine derivatives as potential butyrylcholinesterase inhibitors with vasorelaxation activity", BIOORGANIC & MEDICINAL CHEMISTRY LETTERS, PERGAMON, AMSTERDAM, NL, vol. 23, no. 11, 10 April 2013 (2013-04-10), pages 3162-3165, XP028535185, ISSN: 0960-894X, DOI: 10.1016/J.BMCL.2013.04.008

## Description

### Technical Field of the Invention

The present invention is related to the new molecules produced by masking acidic groups existing in NSAI drugs to prevent or to reduce the hazards of propionic acid derivatives of NSAI drugs to GI (gastrointestinal) system and their synthetic methods. The products resulting from the said synthetic procedures may act as prodrugs of NSAI drugs. In the scope of the present invention, the prodrugs of the molecules with NSAI activity were synthesized and the behaviour of these molecules in simulated environments was examined. The pro-drugs of these molecules were synthesised by way of starting from these molecules per se.

### Background of the Invention

Today, the use area of NSAI (nonsteroidal anti-inflammatory) drugs is very wide. However, the damages of propionic acid derivatives of NSAI drugs to GI (gastrointestinal) system have been proven by the numerous studies. With the present invention, masking acidic groups existing in NSAI drugs was aimed in order to prevent or reduce the damage to the GI tract.

Analgesic compounds are divided into two categories, i.e. narcotic (opioid) analgesics and non-narcotic analgesics. Non-narcotic analgesics are also called as non-steroidal anti-inflammatory (NSAI) or simply anti-inflammatory drugs because of their pharmacological effect profiles. Helen and co-workers, in their work published in Mini-Reviews in Medicinal Chemistry; 9: 124-139*,* mentioned that the use area of NSAI DRUGS was very wide. However, many side effects may occur after prolonged use of these drugs. These adverse effects are the irritation of the gastrointestinal (GI) region followed by ulcer and ulcerative colitis which do not have exact treatment yet. In this case, the development of nonsteroidal anti-inflammatory drugs which do not harm to the GI tract gains importance.

EP 2418200 A1 relates to phthalimide derivatives of non-steroidal and/or TNF-on modulating anti-inflammatory compounds and aims at minimizing of the complications with NSAIDs. The document provides an alternative way to reduce adverse effects of compounds usually used in the treatment of chronic inflammatory processes by using phthalimide derivatives of NSAIDs.

Most of the pro-drugs of NSAI drugs are prepared by the derivatization of carboxyl group in their structures. In the publications of Bundgaard H. in 1989 entitled "The Double Prodrug Concept and its Applications" (Adv. Drug Deliv. Rev. ; 3: 39-65), and also in 1986 entitled "Design of Prodrugs" (Elsevier, New York, Plenum Press, pp. 49-68) it was mentioned that esters and amides were the most used groups in the pro-drug formulations. This is because they have quite good *in vitro* chemical stabilities and this prolongs the shelf-life of the drug. In addition, these ester and amide derivatives also exhibit *in vivo* compliance as they are substrates of enzymes such as esterases and amidases. For example, in a study by Kourounakis et al. in 2000 that is entitled "Reduction of Gastrointestinal Toxicity of NSAIDs via Molecular Modifications Leading to Antioxidant Anti-Inflammatory Drugs" (Toxicology; 144 : 205-210); amide pro-drugs were prepared by way of coupling diclofenac, tolfenamic acid, ibuprofen and indomethacin molecules with cysteamine that is known with antioxidant properties, and the obtained molecules were demonstrated to exhibit both anti-inflammatory and antioxidant effect and to produce much less harm to GI system. The synthesised pro-drugs are given below.

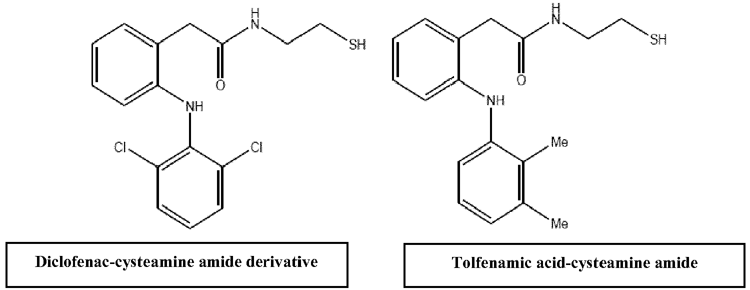

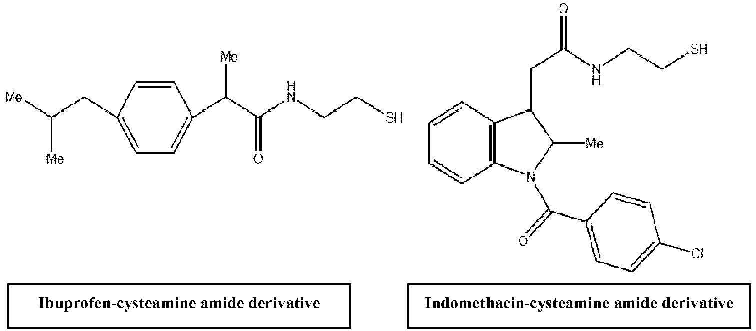

Also, proving the harmful effects of the newly developed NSAI drugs on the GI tract increased the importance of the development of the safer NSAI drugs.

In a study by Mohan R, et al in 2007 entitled "Ester Prodrugs of Flurbiprofen: Synthesis, Hydrolysis and Gastrointestinal Toxicity Plasma" (Indian Journal of Chemistry; Vol. 46B: 1164 to 1168), the researchers synthesized the guiacol ester of ibuprofen molecule with NSAID effect (I) and they examined the toxicological and pharmacological profile of this molecule. At the end of their research, when the gastrointestinal and acute toxicities of the ester molecule were compared with the ibuprofen molecule, it was found out that the toxicities by ester derivatives were much lower and it was observed that they have the same effect in relieving edema and in lowering fever if both molecules are given in equimolar doses.

In another study carried out in 2009 entitled "the ester and amide prodrugs of NSAIDs" (Indian J. Pharm. EDU. Res; 43; 140-149), Lohade AA et al. were prepared the triglyceride derivatives of aspirin (acetylsalicylic acid), being the first effective NSAI drug (II). It was revealed that these aspirin derivatives which consist of medium-chain fatty acid did not cause any damage in the stomach mucosa and they have the same pharmacological effect.

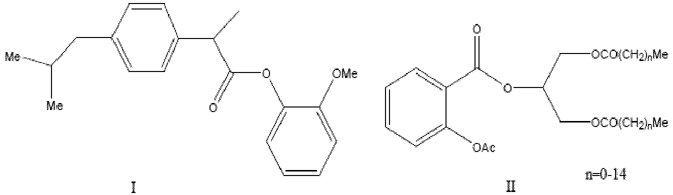

Also in this study, Lohade et al. prepared the ester derivatives by coupling dexketoprofen, ibuprofen and flurbiprofen molecules with simple alcohols (methanol, ethanol etc.). They demonstrated that these readily obtained products underwent enzymatic hydrolysis. In addition, they synthesized the amide prodrugs of these molecules. They concluded with the decision that amide compounds were more stable in the stomach environment.

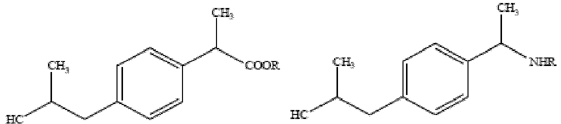

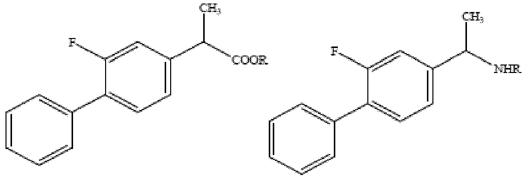

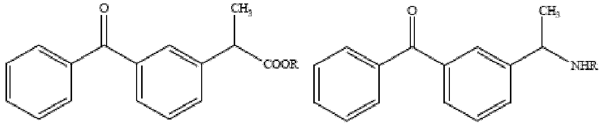

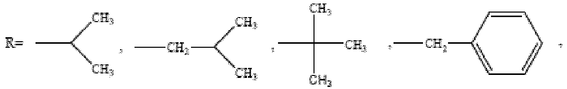

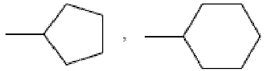

Also, it is known in the prior art that arylpropionic acid class of NSAI drugs, due to their high photoactivities, have a quite sensitive structure and that they present decarboxylation in their carboxylic acid structures. In an article entitled *"*Photochemical transformation of ibuprofen into harmful 4-isobutylacetophenone: Pathways, kinetics, and significance for surface waters" (Water Research 47 (2013) 6109-6121), it was reported that ibuprofen is converted to a harmful product, 4-isobutylacetophenone by photochemical decomposition via decarboxylation. In an investigation entitled *"*Photodegradation of naproxen and its photoproducts in aqueous solution at 254 nm: A kinetic investigation" which was published in the same journal in 2013 (pp. 373-383, volume 47), it was reported that decomposition products in the structure of 1-(6-methoxy-2-naphthyl) ethanol and 2-acetyl-6-methoxynaphthalene were produced by photochemical breakdown of another anti-inflammatory drug naproxen at 254 nm.

In the light of this knowledge, it is an object of this invention to synthesize the amide derivative pro-drugs of these molecules and to examine the stabilities of the resulting compounds in the media which simulate the gastrointestinal system by starting from NSAID molecules selected from the group of dexketoprofen, ibuprofen or flurbiprofen that include a carboxylic acid group. Amide derivatives synthesized by starting from NSAI drugs will be evaluated as pro-drugs which are expected to reduce the toxic effects on the gastrointestinal tract caused by these drugs having a carboxylic acid group. Another aim of the invention is to solve the stability problem arising from the photo-sensitive structure of the above-mentioned arylpropionic acid class of NSAI drugs.

### Detailed Description of the Invention

With the present invention, the masking of acidic groups present in the NSAI drugs was aimed in order to prevent or reduce the damage to the GI tract. To achieve this goal, it was considered that prodrugs of NSAI drugs should be developed. All these reasons prompted the inventors to synthesize the pro-drugs of some molecules with NSAI activity. Within the scope of the present invention, the prodrugs of molecules with NSAI activity were synthesized and behaviours of these molecules in simulated media were examined. The pro-drugs of these molecules were synthesized by starting again from these molecules per se. More particularly, the present invention provides a method for producing amide derivatives of an NSAID selected from the group of dexketoprofen, ibuprofen or flurbiprofen having a carboxylic acid group which method comprises the steps of:
- formation of a methyl ester of the said carboxylic acid group, and
- obtaining an NSAI drug amide derivative by amidization of this methyl ester with trisubstituted methanamine having the following Formula (I):

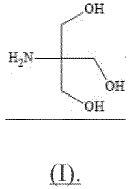

In an exemplary procedure, for instance, firstly the substance with NSAI activity is dissolved in methanol, and then concentrated sulphuric acid was added dropwise, whereby it was reacted at a temperature of about 80°C in a water bath. At the end of about 5-6 hours, the reaction was terminated by checking with TLC, and the ester compounds were obtained by neutralization of the reaction medium. To a part of the resulting ester compounds, it was added equimolar amount of the trisubstituted methanamine compound tris(hydroxymethyl)methanamine, anhydrous potassium carbonate, and an amount of DMSO and these were reacted in an oil bath at a temperature of 40-60°C while mixing. At the end of about 20 hours, the reaction was terminated by controlling with TLC. The solid product obtained after neutralizing the medium, was dried and then purified by crystallization using methyl alcohol or another suitable solvent. The structures of the synthesized compounds were elucidated by elemental analysis (C, H, N, S), ¹H-NMR, IR, UV, and it was confirmed that all the compounds synthesized were in the expected structures. The potential hydrolysis of synthesized pro-drugs were examined at pH 1.2 and pH 6.8 at a temperature of 37±0.5°C, which simulates the gastrointestinal medium. These analysis were carried out using High Pressure liquid Chromatography (HPLC).

The invention mentioned herein may be applied to the synthesis of the following compounds;

| **NSAI Drug** | **Chemical structure of the derivative** | **Chemical name** |
|---|---|---|
| Benoxaprofen | 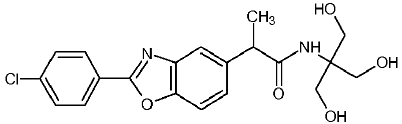 | 2-[2-(4-chlorophenyl)-1,3-benzoxazole-5-yl]-*N*-[1,3-dihydroxy-2-(hydroxymethyl)propan-2-yl]propanamide |
| Diflunisal | 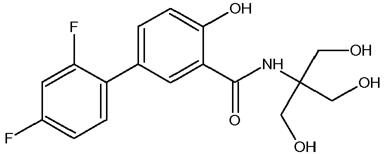 | *N*-[1,3-dihydroxy-2-(hydroxymethyl)propan-2-yl]-2',4'-difluoro-4-hydroxybiphenyl-3-carboxamide |
| Diclofenac | 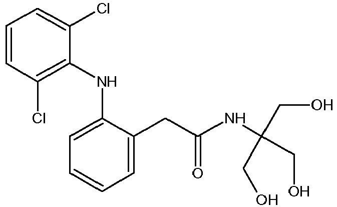 | 2-{2-[(2,6-dichlorophenyl)amino]phenyl}-*N*-[1,3-dihydroxy-2-(hydroxymethyl)propan-2-yl]acetamide |
| Etodolac | 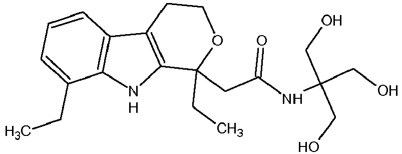 | 2-(1,8-diethyl-1,3,4,9-tetrahydropyrano[3,4-*b*]indole-1-yl)-*N*-[1,3-dihydroxy-2-(hydroxymethyl)propan-2-yl]acetamide |
| Fenbufen | 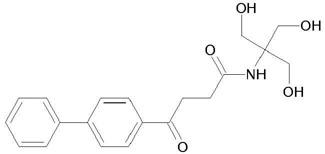 | 4-(biphenyl-4-yl)-*N*-[1,3-dihydroxy-2-(hydroxymethyl)propan-2-yl]-4-oxobutanamide |
| Fenclofenac | 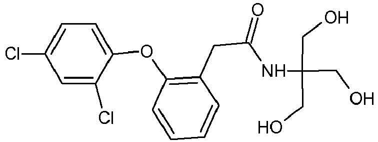 | 2-[2-(2,4-dichlorophenoxy)phenyl]-*N-*[1,3-dihydroxy-2-(hydroxymethyl)propan-2-yl]acetamide |
| Fenoprofen | 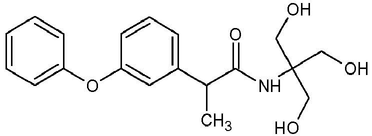 | *N*-[1,3-dihydroxy-2-(hydroxymethyl)propan-2-yl]-2-(3-phenoxyphenyl)propanamide |
| Flunixin | 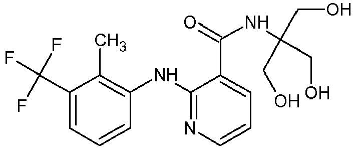 | *N*-[1,3-dihydroxy-2-(hyd roxymethyl)propan-2-yl]-2-{[2-methyl-3-(trifluoromethyl)phenyl]amino}p yridine-3-carboxamide |
| Flurbiprofen | 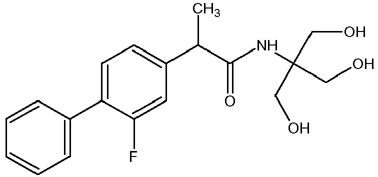 | *N*-[1,3-dihydroxy-2-(hydroxymethyl)propan-2-yl]-2-(2-fluorobiphenyl-4-yl)propanamide |
| Ibufenac | 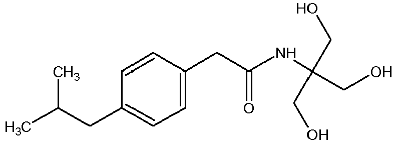 | *N*-[1,3-dihydroxy-2-(hydroxymethyl)propan-2-yl]-2-(4-isobutylphenyl)acetamide |
| Ibuprofen | 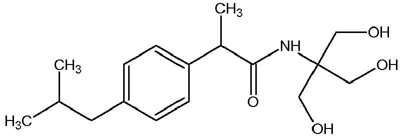 | *N*-[1,3-dihydroxy-2-(hydroxymethyl)propan-2-yl]-2-(4-isobutylphenyl)propanamide |
| Indometazine | 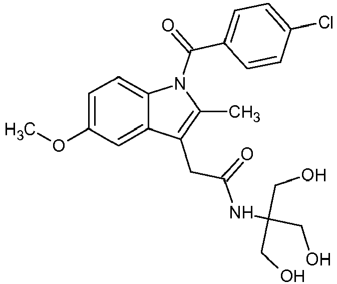 | 2-[1-(4-chlorobenzoyl)-5-methoxy-2-methyl-1*H*-indole-3-yl]-*N*-[1,3-dihydroxy-2-(hydroxymethyl)propan-2-yl]acetamide |
| Indoprofen | 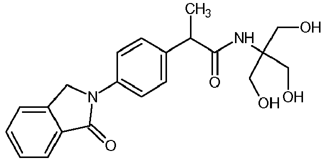 | *N*-[1,3-dihydroxy-2-(hydroxymethyl)propan-2-yl]-2-methyl-2-[4-(1-oxo-2,3-dihydro-1*H*-isoindole-2-yl)phenyl]acetamide |
| Ketoprofen | 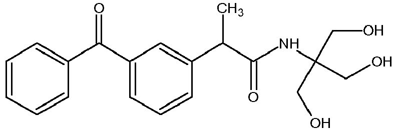 | (±)-2-(3-benzoylphenyl)-*N*-[1,3-dihydroxy-2-(hydroxymethyl)propan-2-yl]propanamide |
| Dexketoprofen | 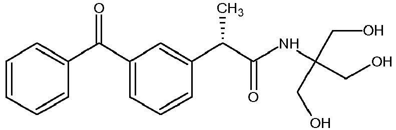 | (+)-(2*S*)-2-(3-benzoylphenyl)-*N*-[1,3-dihydroxy-2-(hydroxymethyl)propan-2-yl]propanamide |
| (-)-Ketoprofen | 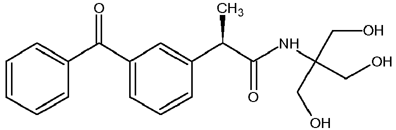 | (-)-(2*R*)-2-(3-benzoylphenyl)-*N*-[1,3-dihydroxy-2-(hydroxymethyl)propan-2-yl]propanamide |
| Ketorolac | 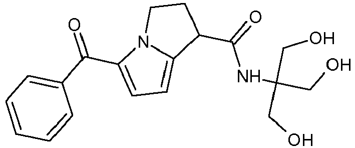 | 5-benzoyl-*N*-[1,3-dihydroxy-2-(hydroxymethyl)propan-2-yl]-2,3-dihydro-1*H*-pyrollizine-1-carboxamide |
| Klidanac | 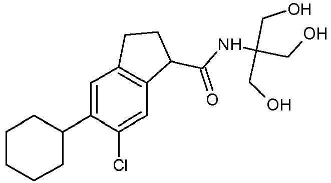 | 6-chloro-*N*-[1,3-dihydroxy-2-(hydroxymethyl)propan-2-yl]-5-cyclohekzyl-2,3-dihydro-1*H-*indene-1-carboxamide |
| Clonixin | 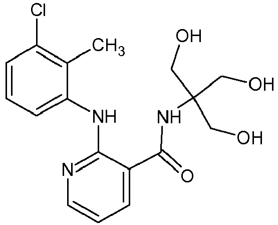 | 2-[(3-chloro-2-methylphenyl)amino]-*N*-[1,3-dihydroxy-2-(hydroxymethyl)propan-2-yl]pyridine-3-carboxamide |
| Clopirac | 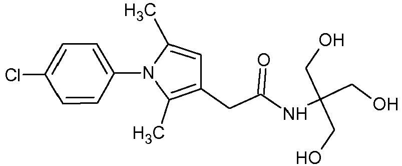 | 2-[1-(4-chlorophenyl)-2,5-dimethyl-1*H*-pyrrole-3-yl]-*N-*[1,3-dihydroxy-2-(hydroxymethyl )propan-2-yl]acetamide |
| Mefenamic acid | 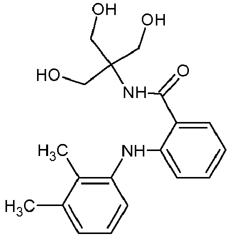 | *N*-[1,3-dihydroxy-2-(hydroxymethyl)propan-2-yl]-2-[(2,3-dimethylphenyl)amino]benzami de |
| Meclofenamic acid | 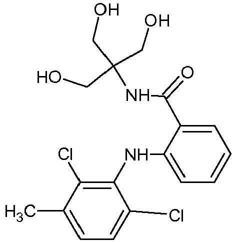 | 2-[(2,6-dichloro-3-methylphenyl)amino]-*N*-[1,3-dihydroxy-2-(hydroxymethyl)propan-2-yl]benzamide |
| Mesalazine | 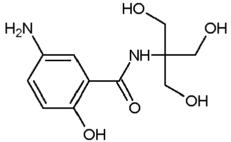 | 5-amino-*N*-[1,3-dihydroxy-2-(hydroxymethyl)propan-2-yl]-2-hydroxybenzamide |
| Niflumic acid | 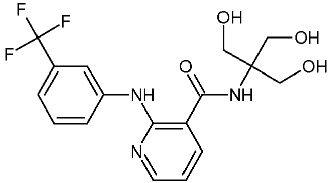 | *N*-[1,3-dihydroxy-2-(hydroxymethyl)propan-2-yl]-2-{[3-(trifluoromethyl)phenyl]amino} pyridine-3-carboxamide |
| Oxaprozine | 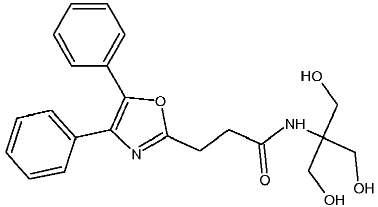 | 3-(4,5-diphenyl-1,3-oxazole-2-yl)-*N*-[1,3-dihydroxy-2-(hydroxymethyl)propan-2-yl]propanamide |
| Olsalazine | 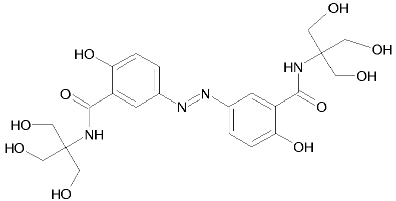 | 3,3'-Diazene-1,2-diylbis{6-hydroxy-*N*-[2-hydroxy-1,1-bis(hydroxymethyl)ethyl] benzamide} |
| Pirprofen | 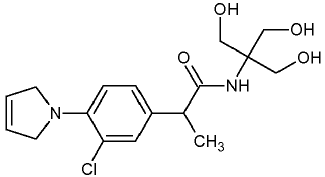 | 2-[3-chloro-4-(2,5-dihydro-1*H-*pyrrole-1-yl)phenyl]-*N*-[1,3-dihydroxy-2-(hydroxymethyl)propan-2-yl]propanamide |
| Sulfasalazine | 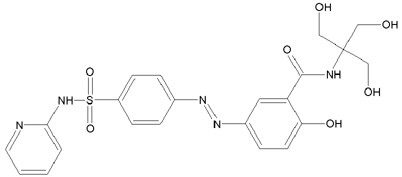 | 2-hydroxy-*N*-[1,3-dihydroxy-2-(hydroxymethyl)propan-2-yl]-5-({4-[(pyridine-2-ylamino)sulfonyl]phenyl}diazen yl)benzamide |
| Sulindac | 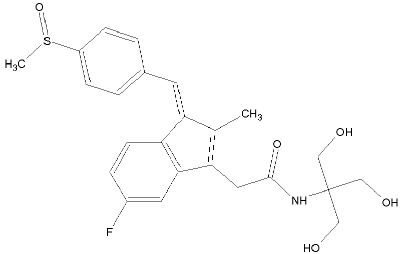 | 2-{(1*Z*)-5-fluoro-2-methyl-1-[4-(methylsulfinyl)benzylidene]-1*H*-indene-3-yl}-*N*-[1,3-dihydroxy-2-(hydroxymethyl)propan-2-yl]acetamide |
| Suprofen | 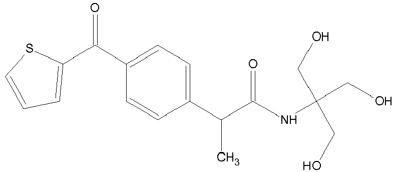 | *N*-[1,3-dihydroxy-2-(hydroxymethyl)propan-2-yl]-2-[4-(2-thienylcarbonyl)phenyl]propan amide |
| Tiaprofenik acid | 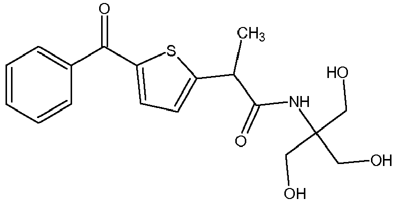 | 2-(5-benzoyl-2-thienyl)-*N*-[1,3-dihydroxy-2-(hydroxymethyl)propan-2-yl]propanamide |
| Tolfenamic acid | 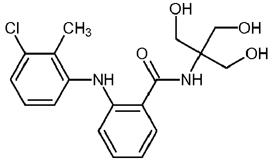 | 2-[(3-chloro-2-methylphenyl)amino]-*N*-[1,3-dihydroxy-2-(hydroxymethyl)propan-2-yl]benzamide |
| Tolmetin | 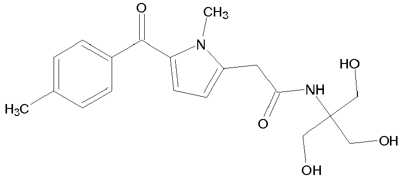 | *N*-[1,3-dihydroxy-2-(hydroxymethyl)propan-2-yl]-2-[1-methyl-5-(4-methylbenzoyl)-1*H*-pyrrole-2-yl]acetamide |
| Zomepirac | 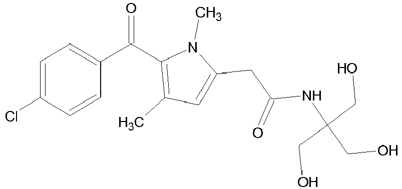 | 2-[5-(4-chlorobenzoyl)-1,4-dimethyl-1 *H*-pyrrole-2-yl]-*N-*[1,3-dihydroxy-2-(hydroxymethyl)propan-2-yl]acetamide |

In the above Table "Indometazine", "Klidanac", "Oxaprozine" and "Thiaprofenik acid" should read "Indometacin", "Clidanac, "Oxaprozin" and "Thaprofenic acid", respectively.

### General Procedures of Synthesis

### Synthesis of methyl ester of dexketoprofen:

### Methyl (2R)-2-(3-benzoylphenyl)propanoate [1a]

About 15 mmole dexketoprofen is dissolved in methanol. After heating under reflux for half an hour in a water bath, 1 ml of concentrated sulfuric acid is added. The reaction was terminated by heating in a water bath at about 80-90°C and neutralized with 5% sodium bicarbonate solution by controlling the pH. The resulting crude product was extracted into organic phase with ether. The ethereal solution was dried with anhydrous sodium sulfate. The ether was evaporated to yield the crude product.

### Synthesis of amide derivative from methyl ester of dexketoprofen: (+)-(2S)-2-(3-Benzoylphenyl)-N-[1,3-dihydroxy-2-(hydroxymethyl)propan-2-yl]propanamide [1b]

Compound **1a** synthesized in the previous step, trisubstituted methanamine compound (i.e. tris(hydroxymethyl)methanamine) and anhydrous potassium carbonate in equimolar ratios (approximately 9 mmole) in 10 mL of DMSO were stirred in a reflux in an oil bath at about 40-50°C for about 20 hours. The reaction medium is treated with plenty of water and neutralized with 10% HC1. The crude product is precipitated by the addition of sodium chloride. The crude product which is washed with water is purified by crystallization with a suitable solvent after it is dried.

### The synthesis of methyl ester of ibuprofen: Methyl 2-(4-isobutylphenyl)propanoate [2a]

About 25 mmole (5.16 g) ibuprofen was dissolved in methanol. After heating under reflux for half an hour in a water bath, 1 ml of concentrated sulfuric acid was added dropwise. The reaction medium which was heated at 80-90°C in a water bath and the reaction is terminated by checking with TLC and the reaction mixture is neutralized with 5% sodium bicarbonate solution by controlling the pH. The resulting crude product was extracted into organic phase with ether. The ethereal solution is dried with anhydrous sodium sulfate. The crude product is obtained by the evaporation of ether.

### The synthesis of amide derivative from methyl ester of ibuprofen: N-[1,3-dihydroxy-2-(hydroxmethyl)propan-2-yl]-2-(4-isobutylphenyl)propanamide [2b]

The compound **2a** synthesized in the previous step, trisubstituted methanamine compound (i.e. tris(hydroxymethyl)methanamine) and anhydrous potassium carbonate in equimolar ratios (approximately 9 mmole) in 10 mL of DMSO were stirred in a reflux in an oil bath at about 40-50°C for about 20 hours. The reaction medium is treated with plenty of water and neutralized with 10% HCl. The crude product is precipitated by the addition of sodium chloride. The crude product which is washed with water is purified by crystallization with a suitable solvent after it is dried.

### The synthesis of methyl ester of flurbiprofen: Methyl 2-(2-fluorobiphenyl-4-yl)propanoate [3a]

About 30 mmol flurbiprofen was dissolved in methanol. After heating under reflux for half an hour in a water bath, 1 ml of concentrated sulfuric acid was added dropwise. The reaction medium which was heated at 80-90°C in a water bath and the reaction is terminated by checking with TLC and the reaction mixture is neutralized with 5% sodium bicarbonate solution by controlling the pH. After it was neutralized, it was kept in the fridge together with the medium in which it was neutralized. The precipitated solid compound is collected by filtration. The obtained crude product was purified by crystallization with a suitable solvent. If it has an enough purity, it is used in the next step.

### The synthesis of amide derivative from methyl ester of flurbiprofen: N-[1,3-Dihydroxy-2-(hydroxymethyl)propan-2-yl]-2-(2-fluorobiphenyl-4-yl)propanamide [3b]

The compound **3a** synthesized in the previous step, trisubstituted methanamine compound (i.e. tris(hydroxymethyl)methanamine) and anhydrous potassium carbonate in equimolar ratios (approximately 9 mmole) in 10 mL of DMSO were stirred in a reflux in an oil bath at about 40-50°C for about 20 hours. The reaction medium is treated with plenty of water and neutralized with 10% HCl. The crude product is precipitated by the addition of sodium chloride. The crude product which is washed with water is purified by crystallization with a suitable solvent after it is dried.

### Chromatographic Studies

### Thin Layer Chromatography

During the course of synthetic studies, the reaction phases and time, the purity of the final product was determined by Thin Layer Chromatography (TLC). Samples taken from the reaction medium or the final product were controlled by using commercially available 0.2 mm silica gel F-254 (Merck) plates as adsorbent and petroleum ether-ethyl acetate (50:50) as solvent system. The saturation of the medium was achieved after the stated solvent system was added into the chromatography tank. Our compounds dissolved in methanol were applied to the TLC plate. The compounds were developed at 25°C and their Rf values were determined. The spots belonging to the compounds were examined under UV light at 254 nm and the colored spots thus obtained were marked.

### High-Pressure Liquid Chromatography

The purity of the final product during the synthesis was determined by High Pressure Liquid Chromatography (HPLC). In Reverse-Phase High Pressure Liquid Chromatography (RP-HPLC) method; an Agilent 1100 Series High Pressure Liquid Chromatography apparatus, a Waters Nova-Pak C18 (3.9x150 mm) column, a G 1311 Quat pump, a G 1315 DAD detector, a HP Vectra VL-DOO DT computer with a software program of Chem Station A-8.3 [847] - 2000 and an Hewlett Packard Deskjet 840C printer were used.

During the course of the HPLC studies, 3 different mobile phase systems were used. The information for these systems are given below.
**Mobile Phase-S₁:** A gradient flow was used. The ratios of mobile phases according to the time are given in Table 1.

**Table 1. The ratios of S₁ mobile phase composition according to the time**

| **Time (min)** | **ACN:Buffer** |
|---|---|
| 0-4 | 32.5 : 67.5 |
| 4-6 | 40:60 |
| 6-8 | 47.5:52.5 |
| 8-10 | 40:60 |
| 10-12 | 32.5:67.5 |

**Mobile Phase- S₂:** Mobile phase compositions and ratio ACN : Buffer (45:55)
**Mobile Phase- S₃:** Mobile phase compositions and ratio ACN : Buffer (40:60)

The stock solutions which were prepared by dissolving the synthesized and purified compounds in methanol were diluted into the target concentration (10 µg/ml) with the mobile phase. The solutions were injected into the system in a volume of 50 µl by means of an Hamilton injector at a flow rate of 1 ml/min of the mobile phase.

The peaks belonging to the compounds were examined with a DAD detector at a wavelength of 210 nm
The retention times of the compounds were determined (Table 2).

**Table 2: The retention times of the synthesized compounds (Rt).**

| **The compound code** | **Rf** | **Rt (min)** | **Mobile Phase (HPLC)** |
|---|---|---|---|
| **Dexketoprofen** | | 8.50 | **S₁** |
| **la** | 0.754 | 15.75 | **S₁** |
| **lb** | 0.035 | 3.20 | **S₁** |
| **Ibuprofen** | | 9.11 | **S₂** |
| **2a** | 0.626 | 16.56 | **S₂** |
| **2b** | 0.069 | 2.62 | **S₂** |
| **Flurbiprofen** | | 6,48 | **S₃** |
| **3a** | 0.915 | 12.67 | **S₃** |
| **3b** | 0.050 | 3,03 | **S₃** |

### Hydrolysis Experiment

### Preparation of Mobile Phase Systems

**pH 3.22 Buffer:** 2 ml triethylamine was added into 1000 ml with ultra-distilled water. The pH is adjusted to 3.22 with orthophosphoric acid.
**Injection Volume:** 50 µl
**HPLC Column Type:** ACE C18 reverse phase.
**Flow Rate:** 1 ml/min.
**Flow Rate:** 210 nm

### Mobile Phase Systems

**Mobile Phase - S1** : Gradient flow was used. The ratios of mobile phase compositions according to the time are given in Table 1 in above.
**Mobile Phase - S2 :** The mobile phase composition and their ratio is ACN:Buffer (40:60) and the retention time is 12 min.
**Mobile Phase-S3** : The mobile phase composition and their ratio is ACN:Buffer (40:60) and the retention time is 13 min.

### Preparation of Simulated GI System Media

**pH 1.2 Medium: Hydrochloric acid** (8.5 ml 37%) was added into 1000 ml with water. The pH was controlled (Source: USP).

**pH 6.8 Phosphate Buffer Medium:** 68.05 g of potassium dihydrogen phosphate and 9.2 g of sodium hydroxide are weighed. 10 l of pure water is added on. The pH is checked. If necessary pH is adjusted with ortho-phosphoric acid. (Source: EPA)

### Preparation of the Standard Samples

The amounts of 1 mg from each carboxylic acid, ester and amide compounds are taken, and these were dissolved in 10 mL ACN and stirred in a vortex mixer at 2000 rpm for 1 minute. 100 µL of the sample is taken out of this solution, and diluted with mobile phase to 10 mL (C_{std} = 1 ug/ml)

### Preparation of incubation samples

10 mg of amide compound is dissolved in 2 ml ACN. To this mixture, 88 mL of pH 1.3 or pH 6.8 medium which simulates the GI tract environment was added, and the solution is thoroughly mixed. From this mixture an amount of 15 ml is taken and transferred into the incubation tubes. Samples of 225 µl were taken from the tubes maintained in a water bath at a temperature of 37 ± 0.5 °C at 30, 60, 120 and 240 minutes with a micropipette. The samples taken are transferred to 10 ml test tubes and diluted to 2.5 mL with the mobile phase and stirred with the vortex mixer for 1 minute at 2000 rpm. During the preparation and injection of samples, the samples are kept in ice.

### Degradation Studies

### Preparation of degradation samples

When it was observed during the incubation studies that the synthesized molecules were not hydrolysed at the end of 4 hours (240 min.), it was decided to perform degradation studies in order to be able to see the results of the accelerated conditions.

The samples for degradation studies were prepared by the preparation methods for incubation samples as described above. However, the prepared samples were kept in a water bath at 95 to 100°C for 8 hours. There was no any change in the parameters such as mobile phase types (S₁, S₂ and S₃), column type, flow rate, injection volume.

### Injection Volume: 50 µl

**HPLC Column Type:** ACE C18 reverse phase.
**Flow Rate:** 1 ml/min.
**Wavelength:** 210 nm

### Partition Coefficient Studies

### Preparation of Partition Coefficient Samples

20 mg of amide compound is dissolved in 20 ml of chloroform. 10 mL of this mixture is drawn out (divided into two). This 10 ml of mixture is added to 20 ml with pH 1.2 buffer solution. Likewise the other 10 ml of mixture is added to 20 ml with pH 6.8 buffer solution. The tubes placed in a 37°C incubator are mixed for 24 hours.

After 24 hours the chloroform phase was removed and transferred to separate tubes. The chloroform is evaporated. After evaporation of chloroform, 10 mL of ACN is added and thoroughly mixed. 0.1 ml of the sample is taken out of the mixture with ACN and this sample is added to 10 ml with the mobile phase (S₁, S₂, S₃) (Cn=1ug/ml)

The standard samples used in this study were prepared according to the method of preparation of standard samples given above.

### Injection Volume: 50 µl

**HPLC Column Type:** ACE C18 reverse phase.
**Flow Rate:** 1 ml/min.
**Wavelength:** 210 nm

### Solubility Studies

In order to establish the solubility of the synthesized amide derivative compounds in chloroform and water, each of the amide derivative is weighed in an amount of 10 mg, transferred to a volumetric flask and the solvent is added slowly with a micropipette in a controlled manner until the material dissolved. Studies are conducted at the room temperature.

In the synthesis of amide derivatives of arylpropionic acid which constitutes the subject of the invention, 3 different arylpropionic acid molecules with NSAI activity were used as starting materials. The methyl esters [**1a, 2a, 3a**] were prepared by heating these compounds with methanol in a medium of acidified with concentrated sulfuric acid (acidic), and the propanamide derivatives [**1b, 2b, 3b**] were obtained by reacting these esters in the DMSO medium with trisubstituted methanamine such as tris(hydroxymethyl)methanamine and anhydrous potassium carbonate in the hot environment.

The general synthetic method of the compounds obtained are given in Scheme 1.

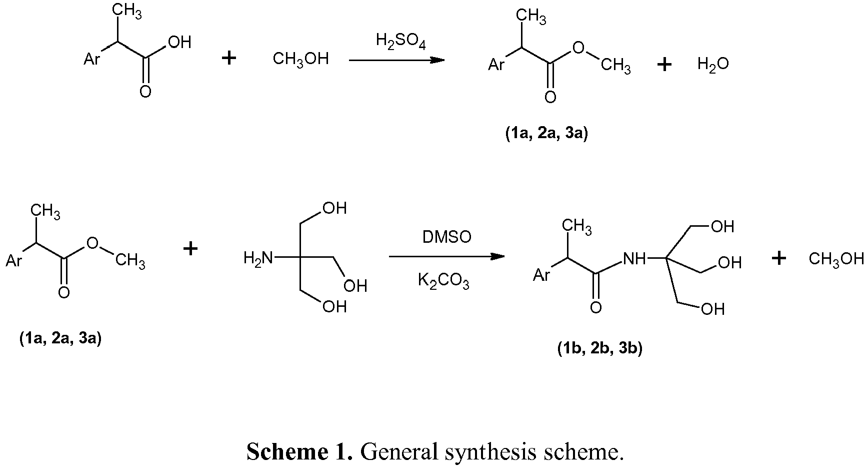

| **Compound code** | **-Ar** |
|---|---|
| **1a, 1b** | 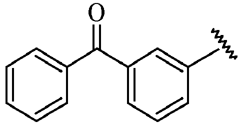 |
| **2a, 2b** | 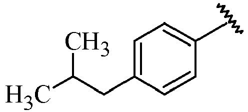 |
| **3a, 3b** | 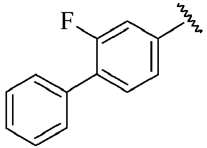 |

### The synthesis of methyl esters obtained by starting from substituted propionic acids (1a, 2a, 3a)

Following the reaction of substituted propionic acids, which are among the main synthetic starting materials of our study, with methanol in an acidic medium at a temperature of 80-90°C under reflux, the methyl esters thereof were obtained with a yield of 95 to 50%. 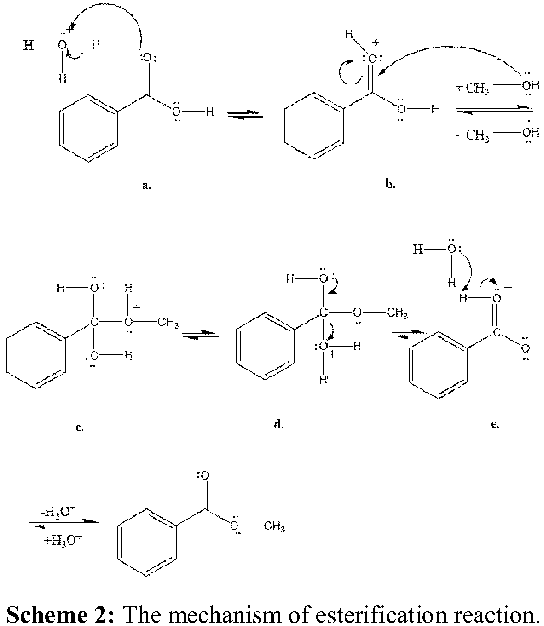
**a.** Carboxylic acid takes a proton from a strong acid catalyst.
**b.** Alcohol gives a smooth tetrahedron intermediate by attacking to the protonated carbonyl group.
**c.** A proton on oxygen atom is lost and it is gained by another oxygen atom.
**d.** The loss of a water molecule gives the protonated ester.
**e.** The transfer of the proton to a base leads to the formation of an ester.

The physical data of the synthesized compounds **1a, 2a** and **3a** are given in Table 3.

**Table 3: The physical data of the compounds 1a, 2a and 3a**

| **Compound Code** | **Compound** | **M.W. (g/mole)** | **Physical state** | **M.P.3 (°C)** | | **Yield (%)** |
|---|---|---|---|---|---|---|
| | | | | **Found** | **Reported (lit.)** | |
| **1a** | 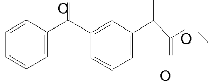 | 268.307 | Pale yellow Viscous Liquid | - | - | 85.3 |
| **2a** | 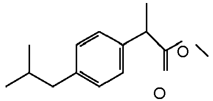 | 220.307 | Colorless Viscous Liquid | - | - | 95.8 |
| **3a** | 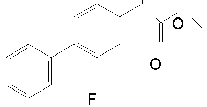 | 258.287 | Solid | 47°C | 46-48°C(43) | 65.0 |

| | | | | | | |
|---|---|---|---|---|---|---|
| ^{a}Melting points uncorrected ^{b}The yields are calculated on purified product. | | | | | | |

During the synthesis of the compounds **1a, 2a and 3a,** no different process is performed and the parameters such as the temperature and the time are the same.

The purity controls of synthesized methyl esters were done by TLC and HPLC methods. When it is worked with S system by TLC, the Rf values obtained at 25°C are given in Table 2. Following the study by HPLC, the retention times of the compounds were determined.

The IR findings belonging to the synthesized **1a, 2a** and **3a** compounds are given in Table 4.

**Table 4 : The IR findings of Compounds 1a, 2a ve 3a.**

| **Compound Code** | **C-H s.b. (ar. str, al. str a.s.b) (cm⁻¹)** | **C=O s.b (cm⁻¹)** | **C=C s.b (ar. str) (cm⁻¹)** | **CH b.b (al. str) (cm⁻¹)** | **C-F s.b (cm⁻¹)** | **C-O s.b (cm⁻¹)** | **CH b.b (ar. str) (cm⁻¹)** |
|---|---|---|---|---|---|---|---|
| **1a** | **3085** | **1730** | **1587** | **1447** | **--** | **1281** | **710** |
| | **2961** | **1657** | | **1435** | | | |
| | **2937** | | | **1398** | | | |
| **2a** | **2964** | **1740** | **1512** | **1460** | **--** | **1203** | **850** |
| | **2880** | | | **1452** | | **1162** | **840** |
| | | | | **1434** | | | |
| **3a** | **3008** | **1733** | **1625** | | **1332** | | |
| | **2987** | | **1581** | **1484** | | **1160** | **764** |
| | **2948** | | **1562** | **1438** | | **1146** | **640** |
| | **2848** | | **1516** | **1415** | | **1131** | |

When the IR spectrums of synthesized **1a, 2a** and **3a** compounds in the scope of the invention were examined, it was determined that the O-H stretching band which exists in the structure of substituted propionic acids being the primary constituent was disappeared. For **1a, 2a** and **3a** compounds, C-H stretching bands at 3085-2848 cm⁻¹, C=O stretching bands at 1740 to 1657 cm⁻¹, C=C stretching bands at 1625-1512 cm⁻¹, C-H bending bands at 1484-1415 cm⁻¹, C=O stretching bands at 1281-1131 cm⁻¹ and C-H bending bands at 850-640 cm⁻¹ were detected (Table 4). It was observed that the obtained findings were in correlation with the literature. In contrast to the starting compounds, C=O stretching band belonging to the ester group of the compounds **1a, 2a** and **3a** was observed at between 1740 to 1730 cm⁻¹ and the compound **3a** exhibited a C-F stretching band at 1332 cm⁻¹ as being different from the others.

### Synthesis of Amide derivatives from methyl esters (1b, 2b, 3b)

The amide derivatives were obtained following the reaction of the synthesized propionic acid methyl esters with equimolar amount of trisubstituted methanamine compound tris(hydroxymethyl)methanamine and anhydrous potassium carbonate in a DMSO medium at about 40 to 50°C in an oil bath under reflux for about 20 hours and the amide derivatives were obtained in 75% - 45% yield. To remove DMSO, the reaction mixture was treated with plenty of water.

The physical data of synthesized **1b**, **2b** and **3b** compounds are given in Table 5.

**Table 5: The physical data for 1b, 2b and 3b compounds.**

| **Compound code** | **Compound** | **MW. (g/mole)** | **Physical state** | **Melting point (°C)** | **Yield* (%)** |
|---|---|---|---|---|---|
| **1b** | 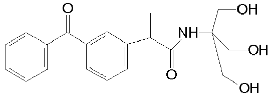 | 357.400 | White solid | 56°C | 45.21 |
| **2b** | 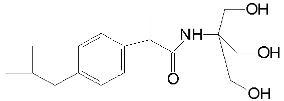 | 309.400 | White pearlescent solid | 114°C | 75.8 |
| **3b** | 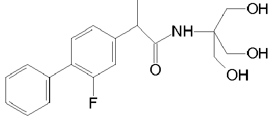 | 347.380 | White pearlescent solid | 173°C | 55.6 |

| | | | | | |
|---|---|---|---|---|---|
| ^{a}Melting points uncorrected ^{b}The yields are calculated on purified product. | | | | | |

Lohade et al. in their study which they performed in 2009 (Indian J. Pharm. Educ. Res; 43 (2): 140-149), synthesized the ester and amide derivatives of ibuprofen, dexketoprofen and flurbiprofen. They carried out the synthesis of amide derivatives by stirring the substituted propionic acid compound in a medium of dichloromethane (DCM) and N,N'-dicyclohexylcarbodiimide (DCC) in a water bath at room temperature followed by the required fractional distillation and crystallization procedures. In their study published in J. Org. Chem; 1999; 64:3554-3556 (1999), Ouari et al. produced a compound with the amide structure by combining the amino acid glycine with tris(hydroxymethyl)methanamine. In this reaction, they reported that they used 2-ethoxy-1-ethoxycarbonyl-1,2-dihydroquinoline as a binder and ethanol as a solvent and that they heated the mixture under reflux.

Djurendic et al., in their study published in J. Serb. Chem. Soc. in. 65: 681-689 (2000), reported that they synthesized some of the ester and amide derivatives of- bis and mono-2-hydroxybenzoic acids and they compared their acidities. They reported that amide synthesis was carried out at 150°C in the presence of a sodium catalyst and they used tris(hydroxymethyl)methanamine as amine in one of the syntheses and the yield was 21%.

D. K., Rajat et al. describes in their study published in Journal of Materials Chemistry; 2010, 20, 7227-7235 two-component charge-transfer interaction mediated organogels (CT-gels) derived from anthracene carboxamides obtained from 2-amino-2 hydroxymethyl-1,3-propanediol (TRIS) which give rise to tris derivatives with 2-4 benzene rings including a naphthalene derivative, and 2,3-dialkoxyanthracenes as donors, with 2,4,7-trinitrofluorenone (TNF) as the common acceptor.

A study which supports the hypothesis that non-steroidal anti-inflammatory drugs (NSAIDs) and antioxidant therapy against the development of Alzheimer's disease discloses ibuprofen covalently linked via an amide bond to lipoic acid. The synthesis of said composition involves the heating of ibuprofen methylester with dialkylamines such as ethylendiamine, 1,4-diaminobutane, and 1,6-diaminohexane, respectively and then the coupling of lipoic acid to aminoamides using HOBT and FDCl as peptide-coupling agents (Sozio, P., D'Aurizio, E., Iannitelli, A., Cataldi, A., Zara, S., Cantalamessa, F., Nasuti, C., Di Stefano, A., Arch. Pharm. Chem. Life Sci. 2010, 343,133-142*).*

The study of Brandl, M. et al. Uournal of Pharmaceutical Sciences, Vol. 84, No. 10, October 1995] investigates methods for improving the stability of ketorolac powder blends under elevated humidity and temperature conditions. The degradation of ketorolac tromethamine in solution give rise to products including decarboxy, 1-keto, and 1-hydroxy analog that are also formed in the solid state whereas in the solid state condensation of ketolorac with tromethamine to form an amide also occurs.

Our method for amide synthesis which we chose in the present invention requires a moderate temperature and a period of about 16 hours, and higher yields was obtained in this procedure compared to the procedures in which tris(hydroxymethyl) methanamine was used.

No different process was performed during the synthesis of **1b**, **2b** and **3b** compounds and the parameters such as temperature and time are the same.

The purity controls of synthesized methyl esters were done by TLC and HPLC methods.

When S system was used in TLC, the Rf values observed at 25°C were given in Table 2. Following the HPLC study, the retention times of the compounds were determined.

IR data for synthesized **1b**, **2b** ve **3b** compounds are given in Table 6.

**Table 6 : IR data for 1b, 2b ve 3b compounds**

| **Compound code** | **O-H and N-H s.b.** (cm⁻¹) | **C-H g.b.** (ar. str., al. str. a.s.b.) (cm⁻¹) | **C=O s.b.** (cm⁻¹) | **C=C s.b.** (ar.str.) (cm⁻¹) | **C-H b.b.** (al.str.) (cm⁻¹) | **C-coN s.b.** (cm⁻¹) | **C-F s.b.** (cm⁻¹) | **C-O s.b.** (cm⁻¹) | **C-H b.b** (ar. str. (cm⁻¹) |
|---|---|---|---|---|---|---|---|---|---|
| **1b** | | | | 1522 | | 1311 | - | | 713 |
| | 3549 | | | | 1450 | 1283 | | 1120 | |
| | 3340 | 2964 | 1666 | | 1433 | 1270 | | 1059 | |
| | 3276 | 2883 | 1620 | | 1418 | 1256 | | 1019 | |
| | 3148 | | | | | 123S | | | |
| **2b** | 3306 | | 1630 | 1546 | | 1374 | - | | |
| | | 2962 | | | | 1367 | | 1121 | |
| | | 2924 | | | 1465 | 1360 | | 1071 | 702 |
| | | 2876 | | | 1452 | 1287 | | 1043 | 696 |
| | | | | | | 1249 | | | |
| **3b** | | 3075 | 1642 | | | 1288 | 1332 | 1046 | |
| | 3312 | 2944 | | 1560 | 1483 | 1254 | | 1023 | 714 |
| | 3246 | 2870 | | 1546 | 1421 | 1221 | | 1010 | 690 |

When the IR spectra for **1b**, **2b** and **3b** compounds which were synthesized in the scope of the invention were examined, it was observed that the O-H stretching band which exists in the structure of substituted propionic acids being the primary constituent and the C=O stretching bond which exists in the methyl ester derivatives were disappeared. For **1b**, **2b** and **3b** compounds, C-H stretching bands at 3549-3148 cm⁻¹, C-H stretching bands at 3075-2870 cm⁻¹, C=O stretching bands at 1666-1620 cm⁻¹, C=C stretching bands at 1560-1522 cm⁻¹, C-H bending bands at 1483-1421 cm⁻¹, C-CO-N stretching bands at 1374-1221 cm⁻¹, **C-O** stretching bands at 1120-1010 at C-H bending bands at 714-690 cm⁻¹ were observed (Table 6). It was observed that the obtained findings were in correlation with the literature. It was determined that the compound **3b** exhibited a C-F stretching band at 1332 cm⁻¹ as in the case of **3a** compound which is an ester form of the former.

The ¹H-NMR spectra of the synthesized compounds confirm the expected structures. The data analysis and the evaluations for these data are given in the following table (Table 7).

**Table 7: ¹H-NMR analysis results for 1b, 2b ve 3b compounds**

| **Compound code** | **¹H-NMR Spectral data (300 MHz), (DMSO-d₆) δ ppm** |
|---|---|
| **1b** | 1,33 (3H, d, Ar-CH**CH₃**); 3,52-3,58 (6H, m, -CH(**CH₂**0H)₃); 3.72-3,78 (1H, q, Ar-**CH**CH₃); 4,75 (3H, t, CH(CH₂**OH**)₃, j₁: 5,7 Hz and j₂: 5,7 Hz ) ; 7,39-7,76 (10 H, m, -CO**NH** and Aromatic ring protons) |
| **2b** | 0,84 (6H, d, Ar-CH₂CH(**CH₃**)₂, j: 6,6 Hz); 1,29 (3H, d, Ar-CH**CH₃,** j;7.2 Hz); 1,68-1,89 (1H, m, Ar-CH₂C**H**(CH₃)₂); 2,38 ( 2H, d, Ar-**CH₂**CH(CH₃)₂, j: 8,7 Hz); 3,48-3,62 (6H, m, - CH(**CH**₂OH)₃); 3,67-3,74 (1H, q, Ar-**CH**CH₃); 4,77 (3H, t, CH(CH₂**OH**)₃, j₁: 5,5 Hz and j₂: 5,5 Hz ); 7,06-7,76 (2H, d, Aromatic ring protons, j: 8,1 Hz), 7,20 (3H, d, -CONH and Aromatic ring protons, j: 8,0 Hz) |
| **3b** | 1,35 (3H, d, Ar-CH**CH₃,** j;6,9 Hz); 3,51-3,60 (6H, m, - CH(**CH**₂OH)₃); 3,81-3,88 (1H, q, Ar-**CH**CH₃); 4,75 (3H, t, CH(CH₂**OH**)₃, j₁: 5,7 Hz and j₂: 5,4 Hz ); 6,99-7,66 (8H, m, Aromatic ring protons). |

The elemental analysis results for **1b**, **2b** and **3b** given as an example confirms the structures of these compounds and the results are given in the following table (Table 8).

**Table 8:**

| Elemental analysis results for **1b**, **2b** and **3b** compounds. | | | | | | |
|---|---|---|---|---|---|---|
| **Compound code** | **C** | | **H** | | **N** | |
| | Calculated | Found | Calculated | Found | Calculated | Found |
| **1b** (+2 H₂O) | 61.06 | 60.17 | 6.92 | 6.235 | 3.56 | 3.458 |
| **2b** | 65.99 | 65.82 | 8.80 | 8.677 | 4.53 | 4.351 |
| **3b** | 65.69 | 64.78 | 6.38 | 5.941 | 4.03 | 4.092 |

### Partition Coefficient (log P) Calculations and Evaluations

The comparison of the experimental and theoretical log P values for **1b**, **2b** ve **3b** compounds and also the theoretical log P values for NSAI compounds which were the starting compounds for the former are given in Table 9.

**Table 9: Log P Values of 1b, 2b ve 3b compounds at pH 1,2 and pH 6,8 media.**

| **Compounds** | **Parameters** | | |
|---|---|---|---|
| | **Log P (Experimental result)** | **Log P (Theoretical)** | **Log P (Theoretical-NSAI derivatives)** |
| **1b (pH 1.2)** | -0.05 | 1.35±0.79 | 2.8±0.33 |
| **1b (pH 6.8)** | +0.05 | | |
| **2b (pH 1.2)** | -0.12 | 2.26±0.77 | 3.72±0.23 |
| **2b (pH 6.8)** | -0.41 | | |
| **3b (pH 1.2)** | -0.14 | 2.65±0.79 | 4.12±0.37 |
| **3b (pH 6.8)** | -0.95 | | |

### Evaluation of Hydrolysis Studies

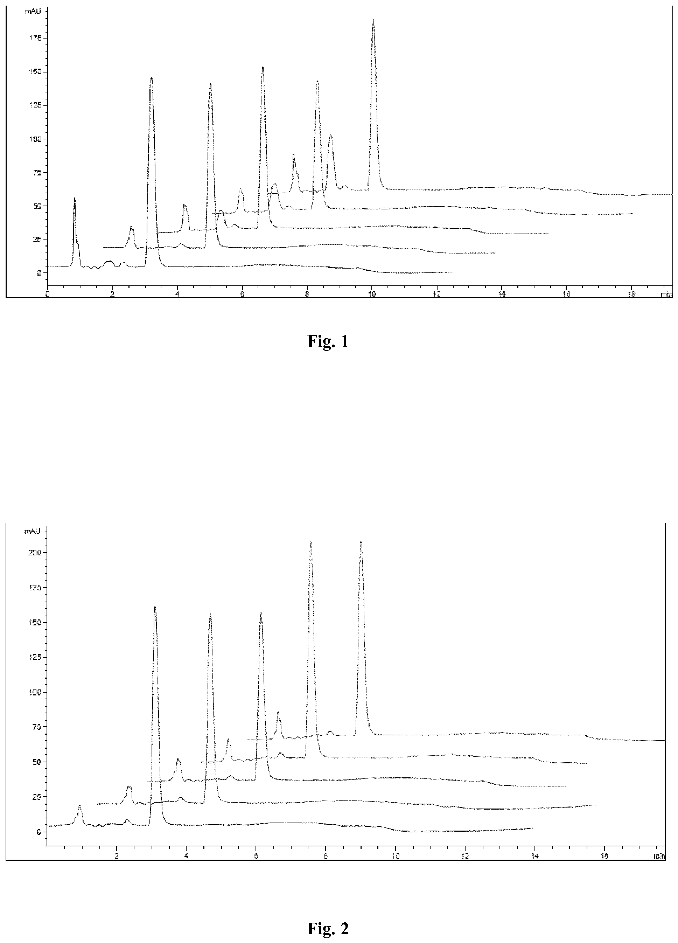

The comparison of HPLC chromatograms of **1b** compound at 0, 30, 60, 120, 180 and 240 mins. in the pH 1.2 medium is given in Figure 1.

The comparison of HPLC chromatograms of **1b** compound at 0, 30, 60, 120, 180 and 240 mins in the pH 6.8 medium is given in Figure 2.

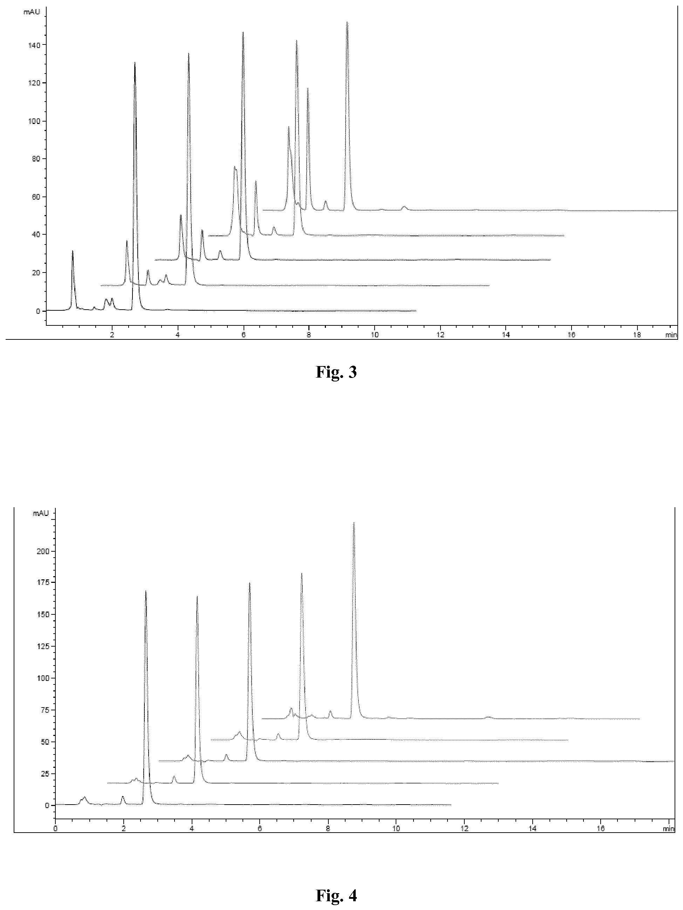

The comparison of HPLC chromatograms of **2b** compound at 0, 30, 60, 120, 180 and 240 mins in the pH 1.2 medium is given in Figure 3.

The comparison of HPLC chromatograms of **2b** compound at 0, 30, 60, 120, 180 and 240 mins in the pH 6.8 medium is given in Figure 4.

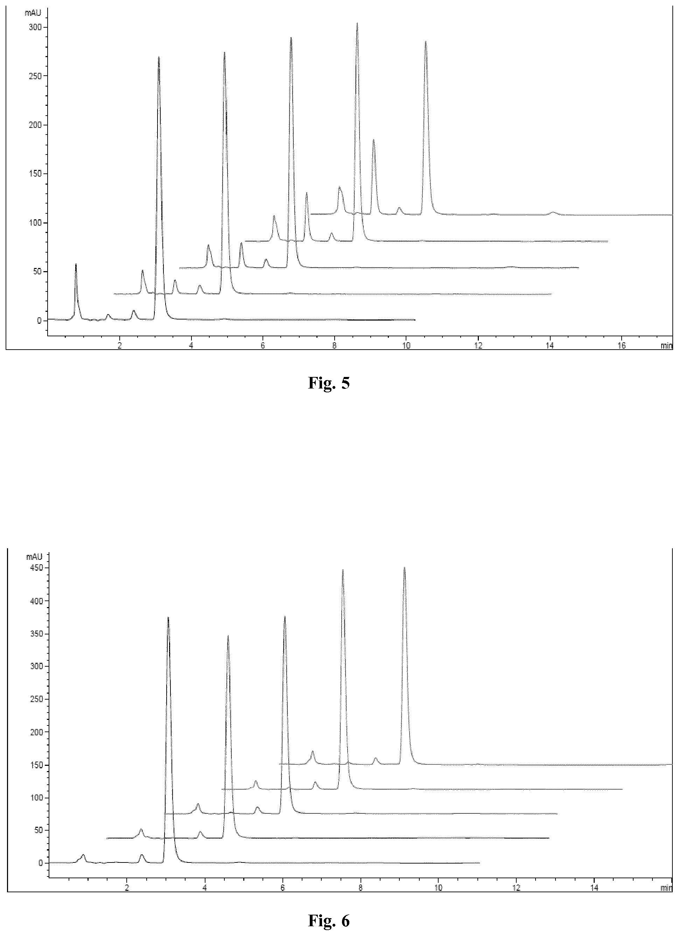

The comparison of HPLC chromatograms of **3b** compound at 0, 30, 60, 120, 180 and 240 mins in the pH 1.2 medium is given in Figure 5.

The comparison of HPLC chromatograms of **3b** compound at 0, 30, 60, 120, 180 and 240 mins in the pH 6.8 medium is given in Figure 5.

Nonsteroidal anti-inflammatory drugs are the best-selling drugs in the world because of their anti-inflammatory cc, antipyretic and analgesic effects. Aspirin is used more commonly because it is also used as an antiplatelet agent. The most prominent adverse effects of these groups are gastrointestinal side effects. To reduce these side effects, numerous studies have been made. For this purpose, the carboxyl groups of the commonly used non-steroidal anti-inflammatory compounds dexketoprofen, ibuprofen and flurbiprofen were masked. For this, the new amide derivatives were synthesized by the methyl esters of the mentioned molecules and their structures were elucidated and their stabilities were examined in buffer media which simulate the GI tract in the buffer media at pH 1.2 and pH 6.8.

In the first stage of the study, the carboxylic acid functional group of non-steroidal anti-inflammatory active compounds was converted to methyl ester thereof (**1a, 2a, 3a**), and then amide derivatives (**1b, 2b, 3b**) were obtained by reacting it with a trisubstituted methanamine compound of a primary amine structure (such as tris(hydroxymethyl) methanamine) was obtained. The structures of these compounds were elucidated by TLC, HPLC, IR, ¹H-NMR and elemental analysis.

The rate of hydrolysis for the synthesized molecules (amide compounds) was followed by HPLC at 37 ± 0.5 °C, at pH 1.2 and pH 6.8 media. As a result of these studies, it was observed that the amide derivatives did not hydrolyse in both media, even after 24 hours. However, the compounds were found to be protonated in an acidic (pH 1.2) medium. It was concluded that the synthesized derivatives were more stable compared to the starting compounds and when other studies are also taken into consideration, it is proposed that GI toxicity of the synthesized compounds will be lower.

The compounds synthesized herein for within the context of the current invention are given below:
2-[2-(4-chlorophenyl)-1,3-benzoxazole-5-yl]-*N*-[1,3-dihydroxy-2-(hydroxymethyl) propan-2-yl]propanamide
*N*-[1,3-dihydroxy-2-(hydroxymethyl)propan-2-yl]-2',4'-difluoro-4-hydroxybiphenyl-3-carboxamide
2-{2-[(2,6-dichlorophenyl)amino]phenyl}-*N*-[1,3-dihydroxy-2-(hydroxymethyl)propan-2-yl]acetamide
2-(1,8-diethyl-1,3,4,9-tetrahydropyrano[3,4-*b*]indole-1-yl)-*N*-[1,3-dihydroxy-2-(hydroxymethyl)propan-2-yl]acetamide
4-(biphenyl-4-yl)-*N*-[1,3-dihydroxy-2-(hydroxymethyl)propan-2-yl]-4-oxobutanamide
2-[2-(2,4-dichlorophenoxy)phenyl]-*N*-[1,3-dihydroxy-2-(hydroxymethyl)propan-2-yl]acetamide
*N*-[1,3-dihydroxy-2-(hydroxymethyl)propan-2-yl]-2-(3-phenoxyphenyl)propanamide
*N*-[1,3-dihydroxy-2-(hydroxymethyl)propan-2-yl]-2-{[2-methyl-3-(trifluoromethyl)phenyl]amino}pyridine-3-carboxamide
*N*-[1,3-dihydroxy-2-(hydroxymethyl)propan-2-yl]-2-(2-fluorobiphenyl-4-il)propanamide
*N*-[1,3-dihydroxy-2-(hydroxymethyl)propan-2-y]-2-(4-isobutylphenyl)acetamide
*N*-[1,3-dihydroxy-2-(hydroxymethyl)propan-2-yl]-2-(4-isobutylphenyl)propanamide
2-[1-(4-chlorobenzoyl)-5-methoxy-2-methyl-1*H*-indole-3-yl]-*N*-[1,3-dihydroxy-2-(hydroxymethyl)propan-2-yl]acetamide
*N*-[1,3-dihydroxy-2-(hydroxymethyl)propan-2-yl]-2-methyl-2-[4-(1-oxo-2,3-dihydro-1*H*-isoindole-2-yl)phenyl]acetamide
2-(3-benzoylphenyl)-*N*-[1,3-dihydroxy-2-(hydroxymethyl)propan-2-yl]propanamide
(2*S*)-2-(3-benzoylphenyl)-*N*-[1,3-dihydroxy-2-(hydroxymethyl)propan-2-yl]propanamide
(2*R*)-2-(3-benzoylphenyl)-*N*-[1,3-dihydroxy-2-(hydroxymethyl)propan-2-yl]propanamide
6-chloro-*N*-[1,3-dihydroxy-2-(hydroxymethyl)propan-2-yl]-5-cyclohexyl-2,3-dihydro-1*H-*indene-1-carboxamide
2-[(3-chloro-2-methylphenyl)amino]-*N*-[1,3-dihydroxy-2-(hydroxymethyl)propan-2-yl] pyridine-3-carboxamide
2-[1-(4-chlorophenyl)-2,5-dimethyl-1*H-*pyrrole-3-yl]-*N-*[1,3-dihydroxy-2-(hydroxymethyl)propan-2-yl]acetamide
*N*-[1,3-dihydroxy-2-(hydroxymethyl)propan-2-yl]-2-[(2,3-dimethylphenyl)amino]benzamide
2-[(2,6-dichloro-3-methylphenyl)amino]-*N-*[1,3-dihydroxy-2-(hydroxymethyl)propan-2-yl] benzamide
5-amino-*N-*[1,3-dihydroxy-2-(hydroxymethyl)propan-2-yl]-2-hydroxybenzamide
*N*-[1,3-dihydroxy-2-(hydroxymethyl)propan-2-yl]-2-{[3-(trifluoromethyl)phenyl] amino}pyridine-3-carboxamide
3-(4,5-diphenyl-1,3-oxazole-2-yl)-*N*-[1,3-dihydroxy-2-(hydroxymethyl)propan-2-yl] propanamide
3,3'-Diazene-1,2-diylbis{6-hydroxy-*N*-[2-hydroxy-1,1-bis(hydroxymethyl)ethyl]benzamide}
2-[3-chloro-4-(2,5-dihydro-1*H-*pyrrol-1-yl)phenyl]-*N-*[1,3-dihydroxy-2-(hydroxymethyl)propan-2-yl]propanamide
2-hydroxy-*N*-[1,3-dihydroxy-2-(hydroxymethyl)propan-2-yl]-5-({4-[(pyridine-2-ylamino) sulfonyl]phenyl} diazenyl)benzamide
2-{(1*Z*)-5-fluoro-2-methyl-1-[4-(methylsulfinyl)benzilidene]-1*H-*indene-3-yl}-*N-*[1,3-dihydroxy-2-(hydroxymethyl)propan-2-yl]acetamide
*N*-[1,3-dihydroxy-2-(hydroxymethyl)propan-2-yl]-2-[4-(2-thienylcarbonyl)phenyl] propanamide
2-(5-benzoyl-2-thienyl)-*N*-[1,3-dihydroxy-2-(hydroxymethyl)propan-2-yl]propanamide
2-[(3-chloro-2-methylphenyl)amino]-*N*-[1,3-dihydroxy-2-(hydroxymethyl)propan-2-yl]benzamide
*N*-[1,3-dihydroxy-2-(hydroxymethyl)propan-2-yl]-2-[I-methyl-5-(4-methylbenzoyl)-1*H*-pyrrol-2-yl]acetamide
2-[5-(4-chlorobenzoyl)-1,4-dimethyl-1*H*-pyrrol-2-yl]-N*-*[1,3-dihydroxy-2-(hydroxymethyl) propan-2-yl]acetamide

## Claims

1. A method for producing amide derivatives of an nonsteroidal anti-inflammatory drug (NSAID) having a carboxylic acid group which is dexketoprofen, ibuprofen or flurbiprofen, comprising the steps of:
- formation of a methyl ester of the said carboxylic acid group, and
- obtaining an NSAI drug amide derivative by amidization of this methyl ester with trisubstituted methanamine having the following Formula (I):

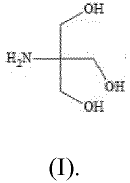

2. A method according to claim 1 comprising the step of obtaining the methyl ester by treating carboxylic acid group with methanol.

3. An amide derivative of an NSAI drug having one of the following formulae:
2-[2-(4-chlorophenyl)-1,3-benzoxazole-5-yl]-*N*-[1,3-dihydroxy-2-(hydroxymethyl) propan-2-yl]propanamide
*N*-[1,3-dihydroxy-2-(hydroxymethyl)propan-2-yl]-2',4'-difluoro-4-hydroxybiphenyl-3-carboxamide
2- {2-[(2,6-dichlorophenyl)amino]phenyl}-*N*-[1,3-dihydroxy-2-(hydroxymethyl)propan-2-yl]acetamide
2-(1,8-diethyl-1,3,4,9-tetrahydropyrano[3,4-*b*]indole-1-yl)-*N*-[1,3-dihydroxy-2-(hydroxymethyl)propan-2-yl]acetamide
4-(biphenyl-4-yl)-*N-*[1,3-dihydroxy-2-(hydroxymethyl)propan-2-yl]-4-oxobutanamide
2-[2-(2,4-dichlorophenoxy)phenyl]-*N*-[1,3-dihydroxy-2-(hydroxymethyl)propan-2-yl] acetamide
*N*-[1,3-dihydroxy-2-(hydroxymethyl)propan-2-yl]-2-(3-phenoxyphenyl)propanamide
*N*-[1,3-dihydroxy-2-(hydroxymethyl)propan-2-yl]-2-{[2-methyl-3-(trifluoromethyl)phenyl]amino}pyridine-3-carboxamide
*N-*[1,3-dihydroxy-2-(hydromethyl)propan-2-yl]-2-yl]-2-(2-fluorobiphenyl-4-yl)propanamide
*N*-[1,3-dihydroxy-2-(hydroxymethyl)propan-2-yl] -2-(4-isobutylphenyl)acetamide
*N*-[1,3-dihydroxy-2-(hydroxymethyl)propan-2-yl]-2-(4-isobutylphenyl)propanamide 2-[1-(4-chlorobenzoyl)-5-methoxy-2-methyl-1*H-*indole-3-yl]-*N-*[1,3-dihydroxy-2-(hydroxymethyl)propan-2-yl]acetamide
*N*-[1,3-dihydroxy-2-(hydroxymethyl)propan-2-yl]-2-methyl-2-[4-(1-oxo-2,3-dihydro-1*H*-isoindole-2-yl)phenyl]acetamide
2-(3-benzoylphenyl)-*N*-[1,3-dihydroxy-2-(hydroxymethyl)propan-2-yl]propanamide (2*S*)-2-(3-benzoylphenyl)-*N*-[1,3-dihydroxy-2-(hydroxymethyl)propan-2-yl]propanamide
(2*R*)-2-(3-benzoylphenyl)-*N*-[1,3-dihydroxy-2-(hydroxymethyl)propan-2-yl]propanamide
6-chloro-*N*-[1,3-dihydroxy-2-(hydroxymethyl)propan-2-yl]-5-cyclohexyl-2,3-dihydro-1*H*-indene-1-carboxamide
2-[(3-chloro-2-methylphenyl)amino]-*N*-[1,3-dihydroxy-2-(hydroxymethyl)propan-2-yl] pyridine-3-carboxamide
2-[1-(4-chlorophenyl)-2,5-dimethyl-1*H-*pyrrole-3-yl]-*N-*[1,3-dihydroxy-2-(hydroxymethyl)propan-2-yl]acetamide
*N*-[1,3-dihydroxy-2-(hydroxymethyl)propan-2-yl]-2-[(2,3-dimethylphenyl)amino]benzamide
2-[(2,6-dichloro-3-methylphenyl)amino]-*N*-[1,3-dihydroxy-2-(hydroxymethyl)propan-2-yl] benzamide
5-amino-*N*-[1,3-dihydroxy-2-(hydroxymethyl)propan-2-yl]-2-hydroxybenzamide
*N*-[1,3-dihydroxy-2-(hydroxymethyl)propan-2-yl]-2-{[3-(trifluoromethyl)phenyl] amino}pyridine-3-carboxamide
3-(4,5-diphenyl-1,3-oxazole-2-yl)-*N*-[1,3-dihydroxy-2-(hydroxymethyl)propan-2-yl] propanamide
3,3'-Diazene-1,2-diylbis{6-hydroxy-*N*-[2-hydroxy-1,1-bis(hydroxymethyl)ethyl]benzamide}
2-[3-chloro-4-(2,5-dihydro-1*H-*pyrrol-1-yl)phenyl]-*N-*[1,3-dihydroxy-2-(hydroxymethyl) propan-2-yl]propanamide
2-hydroxy-*N*-[1,3-dihydroxy-2-(hydroxymethyl)propan-2-yl]-5-({4-[(pyridine-2-ylamino) sulfonyl]phenyl} diazenyl)benzamide
2-{(1*Z*)-5-fluoro-2-methyl-1-[4-(methylsulfinyl)benzilidene]-1*H-*indene-3-yl}-*N*-[1,3-dihydroxy-2-(hydroxymethyl)propan-2-yl]acetamide
*N*-[1,3-dihydroxy-2-(hydroxymethyl)propan-2-yl]-2-[4-(2-thienylcarbonyl)phenyl] propanamide
2-(5-benzoyl-2-thienyl)-*N*-[1,3-dihydroxy-2-(hydroxymethyl)propan-2-yl]propanamide
2-[(3-chloro-2-methylphenyl)amino]-*N*-[1,3-dihydroxy-2-(hydroxymethyl)propan-2-yl]benzamide
*N*-[1,3-dihydroxy-2-(hydroxymethyl)propan-2-yl]-2-[1-methyl-5-(4-methylbenzoyl)-1*H*-pyrrol-2-yl]acetamide
2-[5-(4-chlorobenzoyl)-1,4-dimethyl-I*H*-pyrrol-2-yl]-*N*-[1,3-dihydroxy-2-(hydroxymethyl) propan-2-yl]acetamide

4. An NSAI drug amide derivative according to claim 3 wherein said NSAI drug derivative is 2-(3-benzoylphenyl)-N-[1 ,3-dihydroxy-2-(hydroxymethyl)-propan-2-yl]propanamide having the following general formula [1b]:

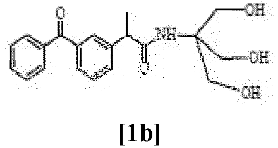

5. An NSAI drug amide derivative according to claim 3 wherein said NSAI drug derivative is *N*-[1,3-dihydroxy-2-hydroxymethyl)propan-2-yl]-2-(4-isobutylphenyl)propanamide having the following general formula [2b]:

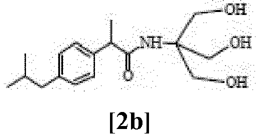

6. An NSAI drug amide derivative according to claim 3 wherein said NSAI drug derivative is *N*-[1,3-Dihydroxy-2-(hydroxymethyl)propan-2-yl]-2-(2-fluorobiphenyl-4-yl) propanamide having the following general formula [3b]:

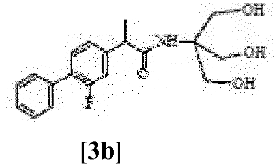

## Patentansprüche

1. Ein Verfahren zur Erzeugung von Amidderivaten eines nichtsteroidalen entzündungshemmenden Medikamentes (NSAID) mit einer carboxylischen Säuregruppe, das Dexketoprofen, Ibuprofen oder Flurbiprofen ist, umfassend die Schritte:
- Bildung eines Methylesters der genannten carboxylischen Säuregruppe, und
- Gewinnen eines Amidderivates eines NSAI-Medikamentes durch Amidierung von diesem Methylester mit trisubstitutiertem Methanamin, das die folgende Formel (I) aufweist:

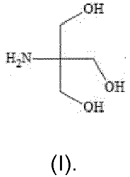

2. Ein Verfahren nach Anspruch 1, umfassend den Schritt des Gewinnens des Methylesters durch Behandeln von carboxylischer Säuregruppe mit Methanol.

3. Ein Amidderivat eines NSAI-Medikamentes, das eine der folgenden Formeln aufweist:
2-[2-(4-Chlorphenyl)-1,3-benzoxazol-5-yl]-*N*-[1,3-dihydroxy-2-(hydroxymethyl)-propan-2-yl]propanamid
*N*-[1,3-Dihydroxy-2-(hydroxymethyl)propan-2-yl]-2',4'-difluor-4-hydroxybiphenyl-3-carboxamid
2-{2-[(2,6-Dichlorphenyl)amino]phenyl}-*N*-[1,3-dihydroxy-2-(hydroxymethyl)-propan-2-yl]acetamid
2-(1,8-Diethyl-1,3,4,9-tetrahydropyran[3,4-*b*]indol-1-yl)-*N*-[1,3-dihydroxy-2-(hydroxymethyl)propan-2-yl]acetamid
4-(Biphenyl-4-yl)-*N*-[1,3-dihydroxy-2-(hydroxymethyl)propan-2-yl]-4-oxobutanamid
2-[2-(2,4-Dichlorphenoxy)phenyl]-*N*-[1,3-dihydroxy-2-(hydroxymethyl)propan-2-yl]-acetamid
*N*-[1,3-Dihydroxy-2-(hydroxymethyl)propan-2-yl]-2-(3-phenoxyphenyl)propanamid
*N*-[1,3-Dihydroxy-2-(hydroxymethyl)propan-2-yl]-2-{[2-methyl-3-(trifluormethyl)-phenyl]amino}pyridin-3-carboxamid
*N*-[1,3-Dihydroxy-2-(hydroxymethyl)propan-2-yl]-2-(2-fluorbiphenyl-4-yl)-propanamid
*N*-[1,3-Dihydroxy-2-(hydroxymethyl)propan-2-yl]-2-(4-isobutylphenyl)acetamid *N*-[1,3-Dihydroxy-2-(hydroxymethyl)propan-2-yl]-2-(4-isobutylphenyl)propanamid
2-[1-(4-Chlorbenzoyl)-5-methoxy-2-methyl-1*H*-indol-3-yl]-*N*-[1,3-dihydroxy-2-(hydroxymethyl)propan-2-yl]acetamid
*N*-[1,3-Dihydroxy-2-(hydroxymethyl)propan-2-yl]-2-methyl-2-[4-(1-oxo-2,3-dihydro-1*H*-isoindol-2-yl)phenyl]acetamid
2-(3-Benzoylphenyl)-*N*-[1,3-dihydroxy-2-(hydroxymethyl)propan-2-yl]propanamid
(2*S*)-2-(3-Benzoylphenyl)-*N*-[1,3-dihydroxy-2-(hydroxymethyl)propan-2-yl]-propanamid
(2*R*)-2-(3-Benzoylphenyl)-*N*-[1,3-dihydroxy-2-(hydroxymethyl)propan-2-yl]-propanamid
6-Chlor-*N*-[1,3-dihydroxy-2-(hydroxymethyl)propan-2-yl]-5-cyclohexyl-2,3-dihydro-1*H*-inden-1-carboxamid
2-[(3-Chlor-2-methylphenyl)amino]-*N*-[1,3-dihydroxy-2-(hydroxymethyl)propan-2-yl]pyridin-3-carboxamid
2-[1-(4-Chlorphenyl)-2,5-dimethyl-1*H*-pyrrol-3-yl]-*N*-[1,3-dihydroxy-2-(hydroxymethyl)propan-2-yl]acetamid
*N*-[1,3-Dihydroxy-2-(hydroxymethyl)propan-2-yl]-2-[(2,3-dimethylphenyl)amino]-benzamid
2-[(2,6-Dichlor-3-methylphenyl)amino]-*N*-[1,3-dihydroxy-2-(hydroxymethyl)propan-2-yl]benzamid
5-Amino-*N*-[1,3-dihydroxy-2-(hydroxymethyl)propan-2-yl]-2-hydroxybenzamid
*N*-[1,3-Dihydroxy-2-(hydroxymethyl)propan-2-yl]-2-{[3-(trifluormethyl)phenyl]-amino }pyridin-3-carboxamid
3-(4,5-Diphenyl-1,3-oxazol-2-yl)-*N*-[1,3-dihydroxy-2-(hydroxymethyl)propan-2-yl]-propanamid
3,3'-Diazen-1,2-diylbis{6-hydroxy-*N*-[2-hydroxy-1,1-bis(hydroxymethyl)ethyl]-benzamid}
2-[3-Chlor-4-(2,5-dihydro-1*H*-pyrrol-1-yl)phenyl]-*N*-[1,3-dihydroxy-2-(hydroxymethyl)propan-2-yl]propanamid
2-Hydroxy-*N*-[1,3-dihydroxy-2-(hydroxymethyl)propan-2-yl]-5-({4-[(pyridin-2-yl-amino)sulfonyl]phenyl}diazenyl)benzamid
2-{(1*Z*)-5-Fluor-2-methyl-1-[4-(methylsulfinyl)benzyliden]-1*H*-inden-3-yl}-*N*-[1,3-dihydroxy-2-(hydroxymethyl)propan-2-yl]acetamid
*N*-[1,3-Dihydroxy-2-(hydroxymethyl)propan-2-yl]-2-[4-(2-thienylcarbonyl)phenyl]-propanamid
2-(5-Benzoyl-2-thienyl)-*N*-[1,3-dihydroxy-2-(hydroxymethyl)propan-2-yl]propanamid
2-[(3-Chlor-2-methylphenyl)amino]-*N*-[1,3-dihydroxy-2-(hydroxymethyl)propan-2-yl]benzamid
*N*-[1,3-Dihydroxy-2-(hydroxymethyl)propan-2-yl]-2-[1-methyl-5-(4-methylbenzoyl)-1*H*-pyrrol-2-yl]acetamid
2-[5-(4-Chlorbenzoyl)-1,4-dimethyl-1*H*-pyrrol-2-yl]-*N*-[1,3-dihydroxy-2-(hydroxymethyl)propan-2-yl]acetamid

4. Ein Amidderivat eines NSAI-Medikamentes nach Anspruch 3, wobei das genannte NSAI-Medikamenten-Derivat 2-(3-Benzoylphenyl)-*N*-[1,3-dihydroxy-2-(hydroxymethyl)-propan-2-yl]propanamid ist, das die folgende allgemeine Formel [1b] aufweist:

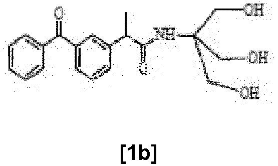

5. Ein Amidderivat eines NSAI-Medikamentes nach Anspruch 3, wobei das genannte NSAI-Medikamenten-Derivat *N*-[1,3-Dihydroxy-2-hydroxymethyl)propan-2-yl]-2-(4-isobutylphenyl)propanamid ist, das die folgende allgemeine Formel [2b] aufweist:

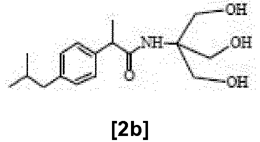

6. Ein Amidderivat eines NSAI-Medikamentes nach Anspruch 3, wobei das genannte NSAI-Medikamenten-Derivat *N*-[1,3-Dihydroxy-2-(hydroxymethyl)propan-2-yl]-2-(2-fluorbiphenyl-4-yl)-propanamid ist, das die folgende allgemeine Formel [3b] aufweist:

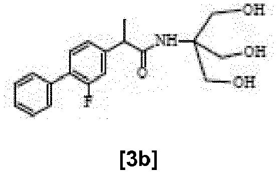

## Revendications

1. Procédé de production de dérivés d'amide d'un anti-inflammatoire non stéroïdien (AINS) ayant un groupe acide carboxylique qui est le dexkétoprofène, l'ibuprofène ou le flurbiprofène, comprenant les étapes suivantes :
- la formation d'un ester de méthyle dudit groupe acide carboxylique, et
- l'obtention d'un dérivé d'amide d'AINS par amidisation de cet ester de méthyle avec une méthanamine trisubstituée ayant la formule (I) suivante :

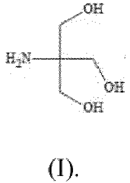

2. Procédé selon la revendication 1, comprenant l'étape d'obtention de l'ester de méthyle en traitant le groupe acide carboxylique avec du méthanol.

3. Dérivé d'amide d'un AINS ayant l'une des formules suivantes :
2-[2-(4-chlorophényl)-1,3-benzoxazole-5-yl]-*N*-[1,3-dihydroxy-2-(hydroxyméthyl)propan-2-yl]propanamide
*N*-[1,3-dihydroxy-2-(hydroxyméthyl)propan-2-yl]-2',4'-difluoro-4-hydroxybiphényl-3-carboxamide
2-{2-[(2,6-dichlorophényl)amino]phényl}-*N*-[1,3-dihydroxy-2-(hydroxyméthyl)propan-2-yl]acétamide
2-(1,8-diéthyl-1,3,4,9-tétrahydropyrano[3,4-*b*]indole-1-yl)-*N*-[1,3-dihydroxy-2-(hydroxyméthyl)propan-2-yl]acétamide
4-(biphényl-4-yl)-*N*-[1,3-dihydroxy-2-(hydroxyméthyl)propan-2-yl]-4-oxobutanamide
2-[2-(2,4-dichlorophénoxy)phényl]-*N*-[1,3-dihydroxy-2-(hydroxyméthyl)propan-2-yl]acétamide
*N*-[1,3-dihydroxy-2-(hydroxyméthyl)propan-2-yl]-2-(3-phénoxyphényl)propanamide
*N*-[1,3-dihydroxy-2-(hydroxyméthyl)propan-2-yl]-2-{[2-méthyl-3-(trifluorométhyl)phényl]amino}pyridine-3-carboxamide
*N*-[1,3-dihydroxy-2-(hydroxyméthyl)propan-2-yl]-2-(2-fluorobiphényl-4-yl)propanamide
*N*-[1,3-dihydroxy-2-(hydroxyméthyl)propan-2-yl]-2-(4-isobutylphényl)acétamide
*N*-[1,3-dihydroxy-2-(hydroxyméthyl)propan-2-yl]-2-(4-isobutylphényl)propanamide
2-[1-(4-chlorobenzoyl)-5-méthoxy-2-méthyl-1*H*-indole-3-yl]-*N*-[1,3-dihydroxy-2-(hydroxyméthyl)propan-2-yl]acétamide
*N*-[1,3-dihydroxy-2-(hydroxyméthyl)propan-2-yl]-2-méthyl-2-[4-(1-oxo-2,3-dihydro-1*H*-isoindole-2-yl)phényl]acétamide
2-(3-benzoylphényl)-*N*-(1,3-dihydroxy-2-(hydroxyméthyl)propan-2-yl]propanamide
(2*S*)-2-(3-benzoylphényl)-*N*-[1,3-dihydroxy-2-(hydroxyméthyl)propan-2-yl]propanamide
(2*R*)-2-(3-benzoylphényl)-*N*-1,3-dihydroxy-2-(hydroxyméthyl)propan-2-yl]propanamide
6-chloro-*N*-[1,3-dihydroxy-2-(hydroxyméthyl)propan-2-yl]-5-cyclohexyl-2,3-dihydro-1*H*-indène-1-carboxamide
2-[(3-chloro-2-méthylphényl)amino]-*N*-[1,3-dihydroxy-2-(hydroxyméthyl)propan-2-yl]pyridine-3-carboxamide
2-[1-(4-chlorophényl)-2,5-diméthyl-1*H*-pyrrole-3-yl]-*N*-[1,3-dihydroxy-2-(hydroxyméthyl)propan-2-yl]acétamide
*N*-[1,3-dihydroxy-2-(hydroxyméthyl)propan-2-yl]-2-[(2,3-diméthylphényl)amino]benzamide
2-[(2,6-dichloro-3-méthylphényl)amino]-*N*-[1,3-dihydroxy-2-(hydroxyméthyl)propan-2-yl]benzamide
5-amino-*N*-[1,3-dihydroxy-2-(hydroxyméthyl)propan-2-yl]-2-hydroxybenzamide
*N*-[1,3-dihydroxy-2-(hydroxyméthyl)propan-2-yl]-2-{[3-(trifluorométhyl)phényl]amino}pyridine-3-carboxamide
3-(4,5-diphényl-1,3-oxazole-2-yl)-*N*-(1,3-dihydroxy-2-(hydroxyméthyl)propan-2-yl]propanamide
3,3'-Diazène-1,2-diylbis{6-hydroxy-*N*-[2-hydroxy-1,1-bis(hydroxyméthyl)éthyl]benzamide}
2-[3-chloro-4(-2,5-dihydro-1*H*-pyrrol-1-yl)phényl]-*N*-[1,3-dihydroxy-2-(hydroxyméthyl)propan-2-yl]propanamide
2-hydroxy-*N*-[1,3-dihydroxy-2-(hydroxyméthyl)propan-2-yl]-5-({4-([(pyridine-2-ylamino)sulfonyl]phényl}diazényl)benzamide
2-{(1Z)-5-f1uoro-2-méthyl-1-[4-(méthylsulfinyl)benzilidène]-1*H*-indène-3-yl}-*N-*[1,3-dihydroxy-2-(hydroxyméthyl)-propan-2-yl]acétamide
*N*-[1,3-dihydroxy-2-(hydroxyméthyl)propan-2-yl]-2-[4-(2-thiénylcarbonyl)phényl]propanamide
2-(5-benzoyl-2-thiényl)-*N*-[1,3-dihydroxy-2-(hydroxyméthyl)propan-2-yl]propanamide
2-[(3-chloro-2-méthylphényl)amino]-*N*-[1,3-dihydroxy-2-(hydroxyméthyl)propan-2-yl]benzamide
*N*-[1,3-dihydroxy-2-(hydroxyméthyl)propan-2-yl]-2-[1-méthyl-5-(4-méthylbenzoyl)-1*H*-pyrrol-2-yl]acétamide
2-[(5-(4-chlorobenzoyl)-1,4-diméthyl-1*H*-pyrrol-2-yl]-*N*-[1,3-dihydroxy-2-(hydroxyméthyl)propan-2-yl]acétamide.

4. Dérivé d'amide d'AINS selon la revendication 3, dans lequel ledit dérivé d'AINS est le 2-(3-benzoylphényl)-N-[1,3-dihydroxy-2-(hydroxyméthyl)-propan-2-yl]propanamide ayant la formule générale [1b] suivante :

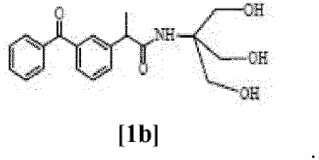

5. Dérivé amide d'AINS selon la revendication 3, dans lequel ledit dérivé d'AINS est le *N*-[1,3-dihydroxy-2-hydroxyméthyl)propan-2-yl]-2-(4-isobutylphényl)propanamide ayant la formule générale [2b] suivante :

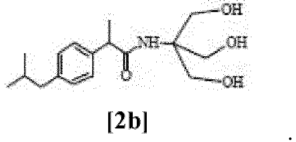

6. Dérivé amide d'AINS selon la revendication 3, dans lequel ledit dérivé d'AINS est le *N*-[1,3-Dihydroxy-2-(hydroxyméthyl)propan-2-yl]-2-(2-fluorobiphényl-4-yl)propanamide ayant la formule générale [3b] suivante :
